# EUROPÄISCHE PATENTSCHRIFT

(11) **EP 1 481 654 B1**
(45) Veröffentlichungstag und Bekanntmachung des Hinweises auf die Patenterteilung: **18.10.2006**
(21) Anmeldenummer: 04009760.2
(22) Anmeldetag: 24.04.2004
(51) Int. Cl.: A61F 2/44

(54) **Zwischenwirbelfusionsimplantat sowie Instrument zur Platzierung und Distraktion des Implantates**
Intervertebral fusion implant and instrument for placement and distraction of said implant
Implant de fusion intervertébrale ainsi que l'instrument pour le placement et la distraction de cet implant

(30) Priorität: 27.05.2003 DE 10324319
(43) Veröffentlichungstag der Anmeldung: 01.12.2004
(73) Patentinhaber: Ulrich GmbH & Co. KG, 89081 Ulm (DE)
(72) Erfinder: Kluger, Patrick, Dr., 89155 Erbach (DE)
(74) Vertreter: Hentrich, Swen

(56) Entgegenhaltungen:
- WO-A-20/04019829
- US-A1- 2002 022 887
- US-A1- 2003 074 063
- US-B1- 6 183 517
- US-B1- 6 419 705

## Beschreibung

Die Erfindung betrifft ein Implantat zur intersomatischen Fusion zweier benachbarter Wirbel, mit einem ersten Implantatteil und einem gegenüber diesem zur Distraktion verstellbaren zweiten Implantatteil.

Implantate zur intersomatischen Fusion zweier Wirbel werden genutzt, um nach einer als notwendig erachteten Resektion einer Bandscheibe das betroffene Segment der Wirbelsäule zu stabilisieren. Bei den Operationsverfahren unterscheidet man hinsichtlich des Zuganges zur Wirbelsäule vom Rücken oder von der Bauchseite her verschiedene Operationstechniken, für die grundsätzlich unterschiedliche Rahmenbedingungen gelten. Bei einem von posterior erfolgenden Zugang ist zu beachten, dass das Rückenmark möglichst schonend aus dem Operationsbereich verschoben wird, die erforderlichen Maßnahmen zur Entfernung der Bandscheibe, Präparation der Wirbelkörper und Einsetzen des Implantats in unmittelbarer Nähe des Rückenmarks erfolgen und der Operateur mit wenig Raum auskommen muss.

Ein Implantat gemäß dem Oberbegriff von Anspruch 1 ist aus der Patentschrift US-B-6183517 bekannt.

Der Erfindung liegt die Aufgabe zugrunde, ein Implantat der eingangs genannten Art so auszubilden, dass bei vollständiger Erhaltung der Distraktionsmöglichkeit und einer einfachen Bedienbarkeit des Implantats zur Distraktion im Operationsfeld in situ die Bauform verkleinert, insbesondere die Länge reduziert werden kann.

Diese Aufgabe wird nach der Erfindung bei einem Implantat der eingangs genannten Art dadurch gelöst, dass das erste Implantatteil und das zweite Implantatteil so gestaltet sind, dass diese sich komplementär im nicht-distrahierten Zustand zu einem in Längsrichtung geteilten Rohr ergänzen, dass am ersten Implantatteil und am zweiten Implantatteil sich radial, in Distraktionsrichtung erstreckende, einander anliegende Führungsflächen ausgebildet sind, und dass Mittel zur Fixierung der relativen Lage der Führungsflächen vorgesehen sind.

Mit einer derartigen Ausführung eines Implantat sind die Vorteile verbunden, dass aufgrund der rohrförmigen Gestaltung des Implantats im Grundzustand sowie der senkrecht zur Rohrachse erfolgenden Distraktion ein sehr stabiles, hoch belastbares Implantat gegeben ist, das bei einer kurzen, aber angemessenen Erstreckung in Längsrichtung nur geringe Abmessungen in Distraktionsrichtung aufweisen kann, so dass zum Einführen des Implantats am Rückenmark vorbei zwischen die Wirbelkörper auch nur ein entsprechend geringer Platzbedarf besteht. Die Führungsflächen stellen dabei sicher, dass bei der Distraktion ein genau definierter Bewegungsablauf bei der Verstellung der beiden Implantatteile relativ zu einander erfolgt, wobei die Führungsflächen zugleich auch genutzt werden, um eine einmal erreichte relative Stellung der beiden Implantatteile dauerhaft zu fixieren, was wiederum die kompakte Bauform des Implantat fördert, da nicht zusätzliche, ausgedehnte Strukturen erforderlich sind zur Verhinderung der Verstellung der beiden Implantatteile relativ zueinander.

Als vorteilhaft hat es sich erwiesen, wenn das Rohr im nicht-distrahierten Zustand eine kreisrunde Querschnittsgestalt besitzt und einen Zylinder bildet. Der Zylinder zeichnet sich durch seine einfach herstellbare, einfache geometrische Form aus, die mit einer hohen Belastbarkeit verbunden ist.

Nach einer besonders bevorzugten Ausführungsform der Erfindung ist vorgesehen, dass die Führungsfläche des ersten Implantatteils an dessen Stirnfläche und die Führungsfläche des zweiten Implantatteils in einem radial verlaufenden Schlitz ausgebildet ist, nämlich an der Innenseite einer Stirnplatte. Dadurch ist erreicht, dass die zur genau definierten Verstellung der beiden Implantatteile sowie zu deren gegenseitiger Fixierung erforderlichen Bauteile sich radial von der Oberfläche des Rohrers nach innen erstrecken und damit beim Einführen des Implantats für diese Bauteile kein erhöhter Platzbedarf erforderlich ist.

Weiterhin im Rahmen der Erfindung besonders bevorzugt ist es, wenn der Schlitz sich radial in Richtung des ersten Implantatteils erweitert, wenn die Stirnplatte zweiteilig mit dem zweiten Implantatteil ausgebildet und in axialer Richtung verstellbar ist, und wenn die Führungsfläche des ersten Implantatteils an einem in den Schlitz eingreifenden Führungskeil mit parallel zu den Schlitzwänden verlaufenden Keilwänden ausgebildet ist. Durch diese Ausgestaltung kann in einfacher Weise mittels Klemmung die relative Lage der beiden Implantatteil zueinander fixiert und auch wieder gelöst werden, wobei durch den Schlitz und durch den Führungskeil bei einer Belastung des Implantats sich eine Verstärkung der Fixierung ergibt, da der Führungskeil in den Schlitz eingepresst wird und mit seinen Keilwänden den Anpressdruck zwischen den Führungsflächen erhöht und somit den Reibschluss verstärkt.

Da aufgrund der geneigten Ausbildung der Schlitzwände und der Keilwände bei einer Verstellung der beiden Implantatteile zueinander in Distraktionsrichtung sich auch eine Verschiebung der beiden Implantatteile zueinander in axialer Richtung ergibt, ist vorgesehen, dass in der Stirnplatte eine axial verlaufende Führungsnut ausgebildet ist zum Zusammenwirken mit einem an dem zweiten Implantatteil ausgebildeten Führungszapfen, da so auch die axiale Verstellung der beiden Implantatteile relativ zueinander in genau definierter Weise erfolgt.

Um eine möglichst große Länge der Führungsfläche zu erreichen, ist im Rahmen der Erfindung vorgesehen, dass der Führungskeil in Umfangsrichtung als einseitig offener Ring gestaltet ist, der mit seiner Ringöffnung im nicht-distrahierten Zustand den Führungszapfen umgreift.

Hinsichtlich eines geringen Platzbedarfes ist es günstig, wenn als Mittel zur Fixierung der relativen Lage des ersten Implantatteils und des zweiten Implantatteils in der Stirnplatte auf der nach innen weisenden Seite eine axial verlaufende Gewindebohrung und an dem zweiten Implantatteils eine Ringöse ausgebildet ist zum Halten einer in die Gewindebohrung eingreifenden Sicherungsschraube. Lediglich durch Verdrehen der Sicherungsschraube in der Gewindebohrung kann die Fixierung der beiden Implantatteile bewirkt oder auch wieder gelöst werden, wobei zu beachten ist, dass zur Betätigung der Mittel zur Fixierung ein Zugang lediglich in axialer Richtung zum Implantat gegeben sein muß, also in der Richtung, in der bereits der Platz vorhanden ist, durch den das Implantat in seine Position eingeführt worden ist.

Der einfache Zugang zur Sicherungsschraube ist erreicht, indem auf der der Stirnfläche gegenüberliegenden Seite eine Implantatöffnung für den Zugang in das Implantatinnere ausgebildet ist.

In der Implantatöffnung sind eine Mehrzahl von Führungnuten ausgebildet, von denen zwei entlang der Längsteilung zwischen dem ersten Implantatteil und dem zweiten Implantatteil verlaufen. Diese Führungsnuten sind dabei vorgesehen, um dem Operateur zur Verstellung der beiden Implantatteile eine Zugriffsmöglichkeit in genau definierter Weise zu bieten.

Wenn in dem freien Ende des Schraubenkopfes der Sicherungsschraube eine Gewindebohrung ausgebildet ist, dann besteht die Möglichkeit, durch Einschrauben eines Gewindestiftes auf das Implantat einwirkende Zugkräfte aufzunehmen und damit bei der Distraktion einer Veränderung der Lage des Implantates insgesamt entgegen zu wirken.

Eine sichere Verankerung des Implantates in die an dieses angrenzende Oberflächen benachbarter Wirbelkörper wird dadurch gefördert, dass die in Distraktionsrichtung sich einander gegenüberliegenden Oberflächen des Rohres eine Verzahnung aufweisen. Vorteilhaft ist es weiterhin, wenn das erste Implantatteil und das zweite Implantatteil aus chirurgischen Stahl und/oder Titan gebildet sind.

Als Mittel zur Fixierung geeignet ist auch alternativ oder ergänzend zwischen das erste Implantatteil und das zweite Implantatteil einzubringender Knochenzement.

Ein zur Platzierung und Distraktion des Implantates besonders geeignetes Instrument ist dadurch gekennzeichnet, dass an einem Handgriff ein hohler Stab angeschlossen ist, der an seinem vorderen freien Ende ein schwenkbares Stellglied aufweist, das zwei Stifte zum Einsetzen in die beiden der Längsteilung zugeordneten Führungsnuten aufweist, dass auf dem hohlen Stab eine Hülse mit einem Schwenkhebel angeschlossen ist, der der Betätigung eines an dem Stellglied zum Schwenken angreifenden Zugstückes dient, und dass in dem hohlen Stab ein hohler Schraubendreher angeordnet ist, den ein Gewindestift zum Einschrauben in die Gewindebohrung des Schraubenkopfes durchgreift. Auf dieses Instrument kann in einfacher Weise das Implantat aufgesetzt werden, indem die Stifte in die Führungsnuten eingesetzt werden und der Schraubendreher zusammen mit dem Gewindestift in Kontakt mit dem Schraubenkopf gebracht wird dergestalt, dass der Gewindestift in die Gewindebohrung eingeschraubt wird zur Aufnahme von Zugkräften und der Schraubendreher in üblicher Weise nur in einem Formschluss dem Schraubenkopf anliegt, um Rotationskräfte auf die Schraube ausüben zu können. Nach dem Lösen der Sicherungsschraube sind die beiden Führungsflächen der beiden Implantatteile gegeneinander verschieblich, wobei die Verschiebung bewirkt wird durch Bedienung des Schwenkhebels, der eine Drehung des Stellgliedes bewirkt, das durch seine zwei Stifte eine Aufspreizung der Führungsnuten senkrecht zur Längsteilung erzeugt mit der daraus resultierenden Verstellung der beiden Implantatteile relativ zueinander. Diese erreichte Verstellung kann erneut fixiert werden durch Betätigung der Sicherungsschraube mittels des Schraubendrehers.

Um eine möglichst große Kontrolle bei der Distraktion zu behalten, ist vorgesehen, dass an dem rückwärtigen Ende des hohlen Stabes ein Stabgewinde ausgebildet ist mit einem darauf zur Begrenzung des Stellweges der Hülse angeordneten Gewindering.

Vorgesehen ist weiterhin, dass das Zugstück an dem oberen Ende des Stellgliedes angreift, dass an dem hohlen Stab im vorderen Bereich an den gegenüberliegenden Stabwänden Nasen ausgebildeten sind, die eine senkrecht zur Längsteilung des Implantats liegende Schwenkachse definieren, und dass das Stellglied mit Abstand zu seiner Verbindung mit dem Zugstück den Nasen anliegt. Auf diese Weise wird mit wenig Platzbedarf durch Ausüben von Zugkräften auf das Stellglied dessen Schwenkbewegung eingeleitet, die in einer in radialer Richtung erfolgende Verstellung der beiden Implantatteile umgesetzt wird.

Weiterhin ist an dem Handgriff ein Bügel gelagert, der zwischen einer den axialen Verstellweg des Gewindestiftes begrenzenden Stellung und einer diese Begrenzung aufhebenden Stellung verschwenkbar ist.

Im folgenden wird die Erfindung an einem in der Zeichnung dargestellten Ausführungsbeispiel näher erläutert; es zeigen:
- Fig. 1: eine perspektivische Darstellung eines erfindungsgemäßen Implantats,
- Fig. 2: eine Seitenansicht des Implantats aus Fig. 1,
- Fig. 3: eine Ansicht der Stirnseite des Implantats aus Fig. 1,
- Fig. 4: eine Draufsicht auf das Implantat gem. Fig. 1,
- Fig. 5: eine Darstellung der Unterseite des Implantats aus Fig. 1,
- Fig. 6: eine der Fig. 1 entsprechenden Darstellung eines distrahierten Implantats,
- Fig. 7: eine der Fig. 2 entsprechende Darstellung des Implantats aus Fig. 6,
- Fig. 8: eine der Fig. 3 entsprechenden Darstellung des Implantates aus Fig. 6,
- Fig. 9: eine der Fig. 5 entsprechende Darstellung des Implantats aus Fig. 6,
- Fig. 10: eine perspektivische Darstellung des isolierten ersten Implantatteils,
- Fig. 11: eine Stirnansicht des ersten Implantatteils aus Fig. 10,
- Fig. 12: eine perspektivische Darstellung des isolierten zweiten Implantatteils,

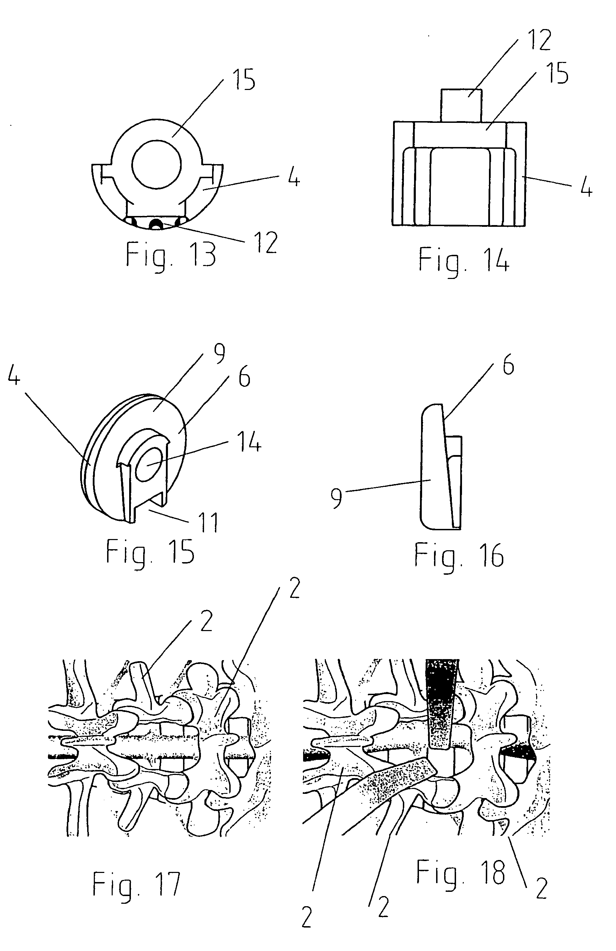

- Fig. 13: eine Stirnansicht des zweiten Implantatteils aus Fig. 12,
- Fig. 14: eine Draufsicht auf das zweite Implantatteil aus Fig. 12,
- Fig. 15: eine perspektivische Darstellung der isolierten Stirnplatte,
- Fig. 16: eine Seitenansicht der Stirnplatte aus Fig. 15,
- Fig. 17: eine schematische Darstellung der Wirbelsäule mit dem Rückenmarkskanal, wie diese sich nach Freilegung dem Operateur bei einem Zugang von posterior darstellt,
- Fig. 18: eine der Fig. 17 entsprechende Darstellung zur Verdeutlichung der Schwierigkeiten zur Schaffung eines angemessenen Platzes während der Operation,
- Fig. 19: eine Seitenansicht eines distrahierten, zwischen zwei Wirbelkörper eingesetzten Implantats,
- Fig. 20: eine Draufsicht auf zwei auf der Deckplatte eines Wirbelkörpers platzierte erfindungsgemäße Implantate,
- Fig. 21: eine der Fig. 19 entsprechende Darstellung mit dem angedeuteten Platzbedarf eines aus dem Stand der Technik bekannten Implantates,
- Fig. 22: eine der Fig. 20 ensprechende Darstellung, in der das obere erfindungsgemäße Implantat ersetzt ist durch eine Andeutung des bei einem aus dem Stand der Technik bekannten Implantats erforderlichen Platzbedarfes,
- Fig. 23: eine Seitenansicht eines mit dem Instrument in den Zwischenwirbelraum eingesetzten Implantats im nicht-distrahierten Zustand, dargestellt ohne Bügel,
- Fig. 24: eine der Fig. 23 entsprechende Darstellung während der Distraktion des Implantats mittels des Instrumentes,
- Fig. 25: eine Draufsicht auf das Implantat und das Instrument aus Fig. 23,
- Fig. 26: eine perspektivische Darstellung des auf das Instrument aufgesetzten Implantats,
- Fig. 27: eine der Fig. 26 ensprechende Darstellung mit dem Bügel in der zweiten Stellung,
- Fig. 28: eine Seitenansicht des auf das Instrument aufgesetzten Implantats,
- Fig. 29: eine der Fig. 28 entsprechende Darstellung eines isolierten Instruments,
- Fig. 30: eine Seitenansicht der Einzelteile eines zerlegten Instruments,

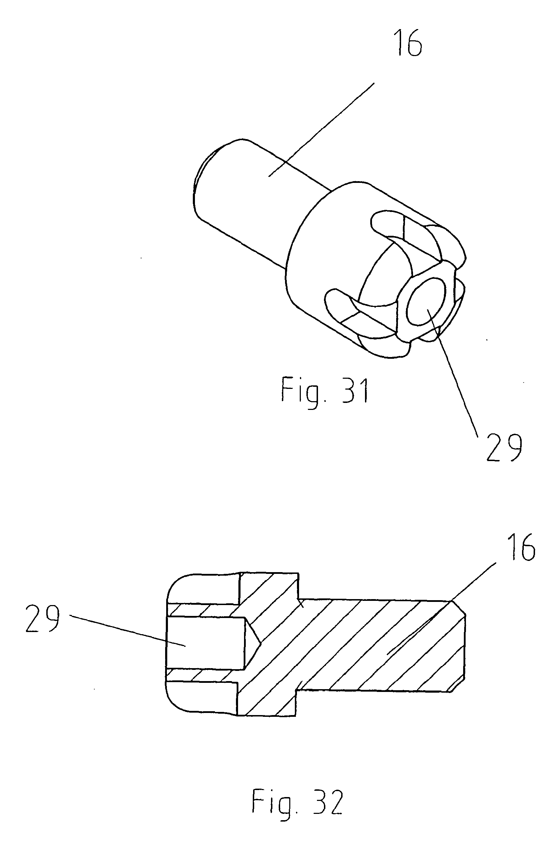

- Fig. 31: eine perspektivische Darstellung der Sicherungsschraube, und
- Fig. 32: einen Längsschnitt durch die Sicherungsschraube aus Fig. 31.

In der Zeichnung dargestellt ist ein Implantat 1, das zur intersomatischen Fusion zweier benachbarter Wirbel 2 verwendet wird, wenn der Zugang zur Wirbelsäule vorzugsweise von posterior erfolgt. Das Implantat 1 besteht aus einem ersten Implantatteil 3 und einem gegenüber diesem zur Distrakion verstellbaren zweiten Implantatteil 4, wobei das erste Implantatteil 3 und das zweite Implantatteil 4 so gestaltet sind, dass diese sich komplementär im nicht-distrahierten Zustand zu einem in Längsrichtung geteilten Rohr 5 von kreisrunder Querschnittsgestalt, also einem Zylinder, ergänzen. Am ersten Implantatteil 3 und am zweiten Implantatteil 4 sind sich radial, nach innen in Distraktionsrichtung erstreckende, einander anliegende Führungsflächen 6 ausgebildet, wobei die Führungsfläche 6 des ersten Implantatteils 3 an dessen Stirnfläche 7 und die Führungfläche 6 des zweiten Implantatteils 4 an einem radial verlaufenden Schlitz 8 an der Innenseite einer Stirnplatte 9 ausgebildet ist, die zweiteilig mit dem zweiten Implantatteil 4 geformt ist. Der Schlitz 8 erweitert sich radial in Richtung des ersten Implantatteils 3; die Führungsfläche 6 des ersten Implantatteils 3 ist an einem in dem Schlitz 8 eingreifenden Führungskeil 10 mit parallel zu den Schlitzwänden verlaufenden Keilwänden ausgebildet. Die Stirnplatte 9 weist eine axial verlaufende Führungsnut 11 auf, die zum Zusammenwirken mit einem an dem zweiten Implantatteil 4 ausgebildeten Führungszapfen 12 vorgesehen ist. Der Führungskeil 10 ist in Umfangsrichtung als einseitig offener Ring gestaltet, der mit seiner Ringöffnung 13 im nicht-distrahierten Zustand den Führungszapfen 12 umgreift.

Das Implantat 1 besitzt weiterhin ein Mittel zur Fixierung der relativen Lage des ersten Implantatteils 3 und des zweiten Implantatteils 4, das eine in der Stirnplatte 9 auf der nach innen weisenden Seite ausgebildete, axial verlaufende Gewindebohrung 14 und eine an dem zweiten Implantatteil 4 ausgebildete Ringöse 15 umfaßt, wobei die Ringöse 15 dazu dient, eine in die Gewindebohrung 14 eingreifende Sicherungsschraube 16 zu halten. Diese Sicherungsschraube 16 ist durch eine Implantatöffnung 17 zugänglich, die auf der der Stirnfläche 7 gegenüberliegenden Seite des Implantates 1 vorhanden ist.

Eine nicht in der Zeichnung dargestellte Alternative nutzt als Mittel zur Fixierung Knochenzement, der zwischen das erste Implantatteil 3 und das zweite Implantatteil 4 eingebracht wird und nach dem Aushärten als massiver Kern das Implantatteil 1 ausfüllt und versteift.

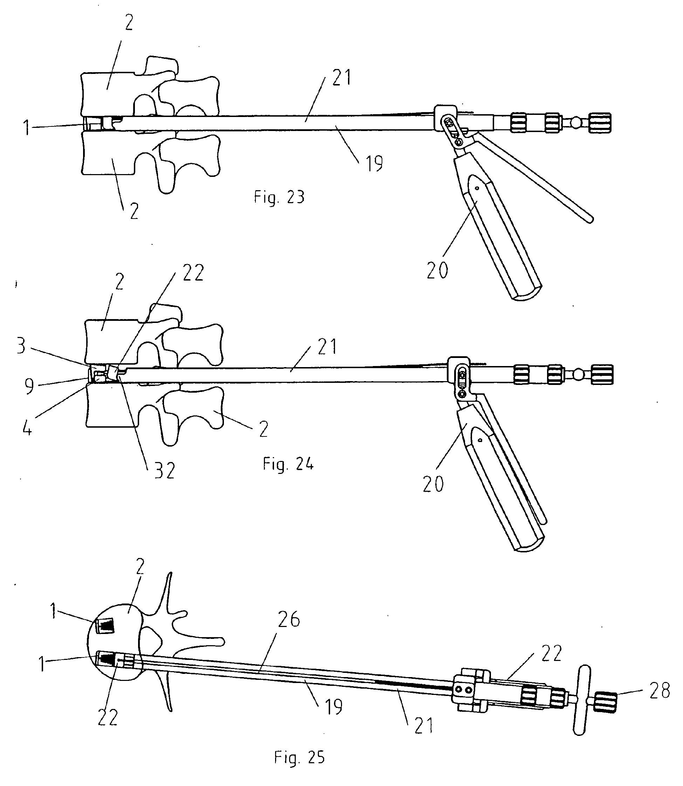

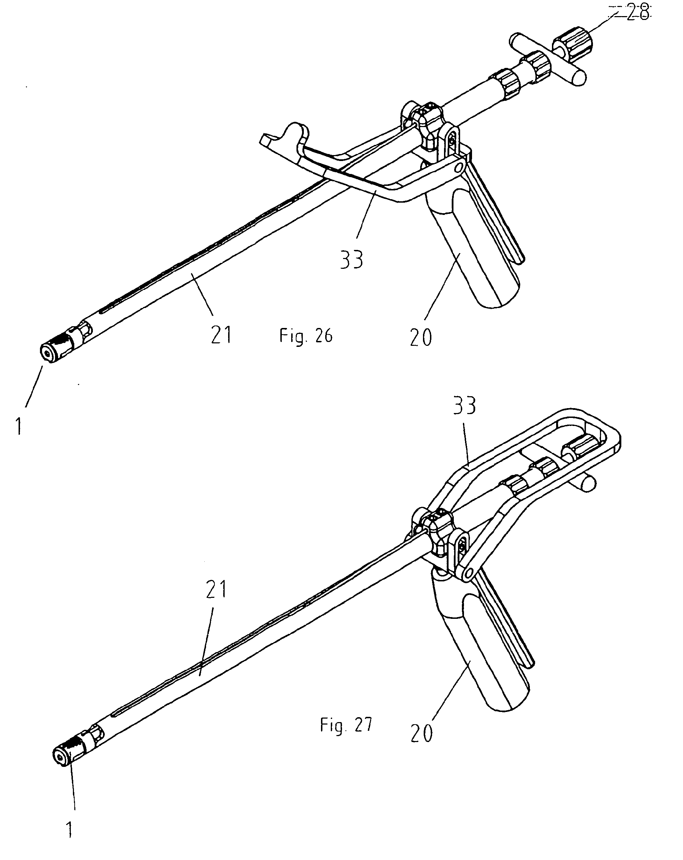

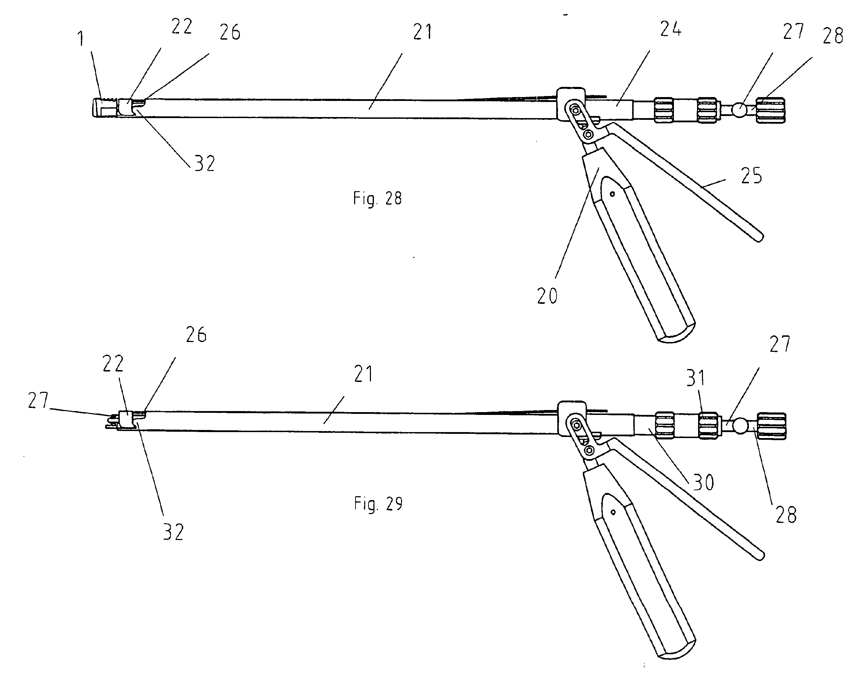

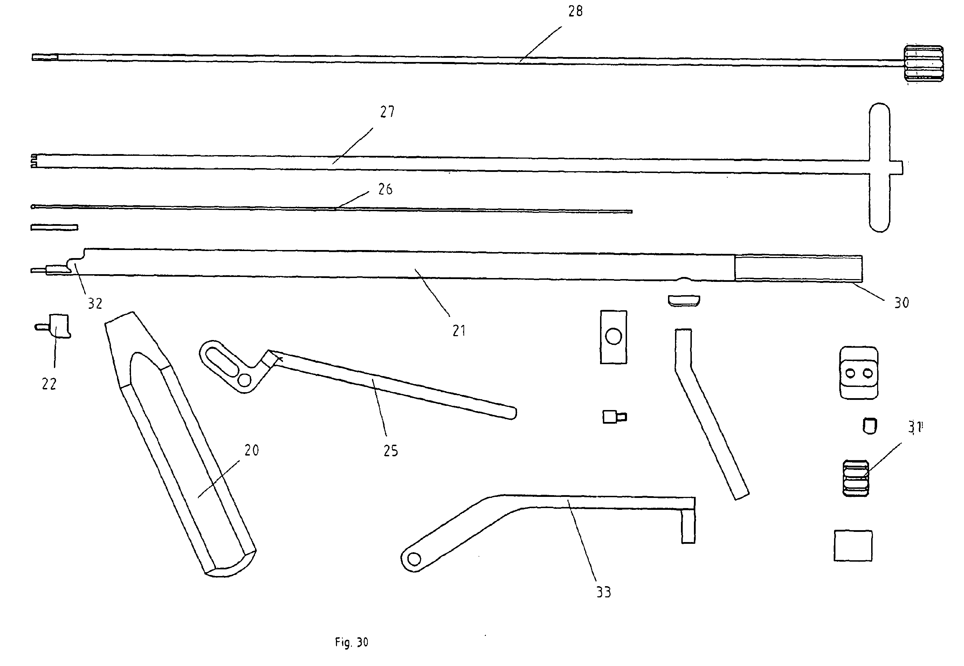

In der Implantatöffnung 17 sind eine Mehrzahl von Führungsnuten 18 ausgebildet, von denen zwei entlang der Längsteilung zwischen dem ersten Implantatteil 3 und dem zweiten Implantatteil 4 verlaufen und dem Zusammenwirken mit einem Instrument 19 dienen, das detalliert in den Fig. 23 bis 30 dargestellt ist.

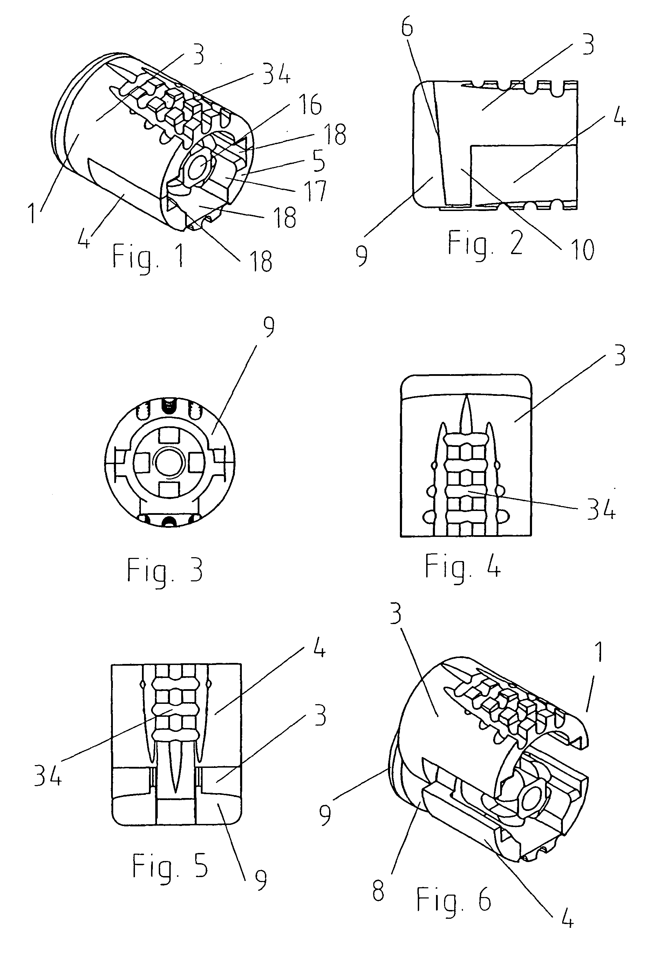

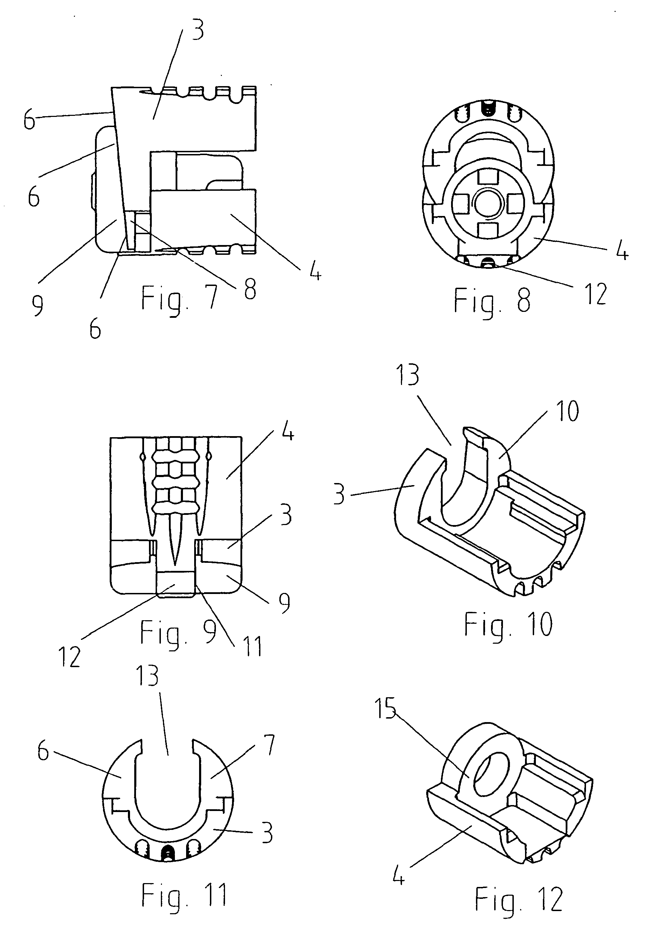

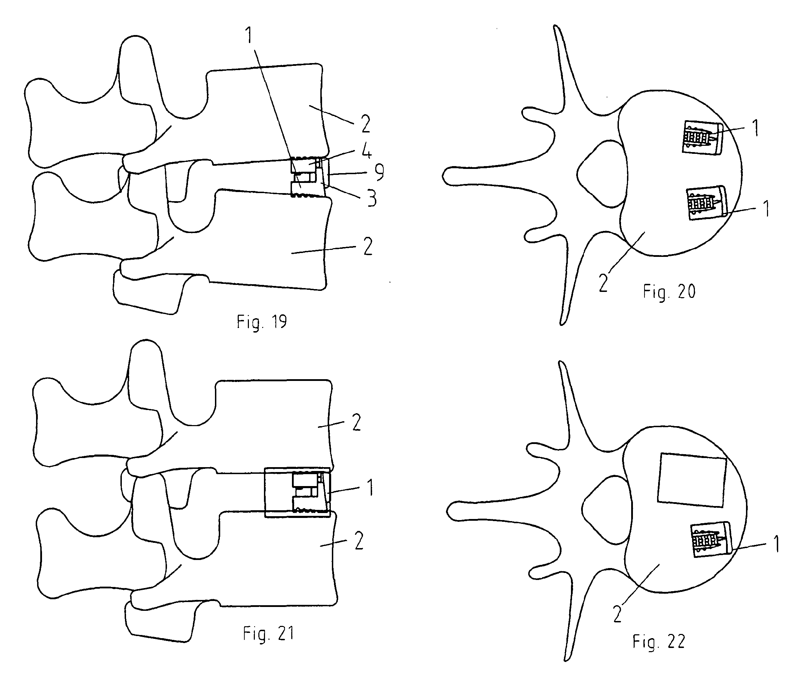

Die Figuren 1 bis 11 und insbesondere der Vergleich mit aus dem Stand der Technik bekannten Implantaten in den Figuren 21 und 22 lassen die kompakte Bauform des erfindungsgemäßen Implantats 1 mit in axialer Richtung kleiner Länge erkennen. Daraus ergibt sich eine vielfältige Verwendungsmöglichkeit des Impantats 1 nicht nur von dorsal oder transforaminal, sondern auch perkutan von weiter lateral beziehungsweise minimalinvasiv von ventral. Die in Figur 20 dargestellte paarweise Verwendung des Implantats 1 ist bevorzugt, aber nicht zwingend, da die Belastbarkeit des Implantats 1 so hoch ist, dass bei entsprechender Indikation und Zugang nur ein Implantat verwendet werden kann.

Das Instrument 19 weist einen an einem Handgriff 20 angeschlossenen hohlen Stab 21 auf, der an seinem vorderen freien Ende ein schwenkbares Stellglied 22 besitzt, das zwei Stifte 23 zum Einsetzen in die beiden der Längsteilung zugeordneten Führungsnuten 18 aufweist, wobei auf dem hohlen Stab 21 eine Hülse 24 mit einem Schwenkhebel 25 angeschlossen ist, der der Betätigung eines an dem Stellglied 22 zum Schwenken angreifenden Zugstückes 26 dient. In dem hohlen Stab 21 ist weiterhin ein hohler Schraubendreher 27 angeordnet, den ein Gewindestift 28 zum Einschrauben in eine Gewindebohrung 29 des Schraubenkopfes der Sicherungsschraube 16 durchgreift. An dem rückwärtigen Ende des hohlen Stabes 21 ist ein Stabgewinde 20 ausgebildet mit einem darauf zur Begrenzung des Stellweges der Hülse 24 angeordneten Gewindering 31. Das Zugstück 26 greift an dem oberen Ende des Stellgliedes 22 an, wobei an dem hohlen Stab 21 im vorderen Bereich an den gegenüberliegenden Stabwänden Nasen 32 ausgebildet sind, die eine senkrecht zur Längsteilung des Implantates 1 liegende Schwenkachse definieren, wobei das Stellglied 22 mit Abstand zu seiner Verbindung mit dem Zugstück 26 den Nasen anliegt. An dem Handgriff 20 ist ein Bügel 33 gelagert, der zwischen einer den axialen Verstellweg des Gewindestiftes begrenzenden Stellung und eine diese Begrenzung aufhebenden Stellung verschwenkbar ist.

## Patentansprüche

1. Implantat zur intersomatischen Fusion zweier benachbarter Wirbel (2), mit einem ersten Implantatteil (3) und einem gegenüber diesem zur Distraktion verstellbaren zweiten Implantatteil (4), wobei das erste Implantatteil (3) und das zweite Implantatteil (4) so gestaltet sind, daß diese sich komplementär im nicht-distrahierten Zustand zu einem in Längsrichtung geteilten Rohr (5) ergänzen, **dadurch gekennzeichnet, daß** am ersten Implantatteil (3) und am zweiten Implantatteil (4) sich radial, in Distraktionsrichtung erstreckende, einander anliegende Führungsflächen (6) ausgebildet sind, und daß Mittel zur Fixierung der relativen Lage der Führungsflächen (6) vorgesehen sind.

2. Implantat nach Anspruch 1, **dadurch gekennzeichnet, daß** das Rohr (5) im nicht-distrahierten Zustand eine kreisrunde Querschnittsgestalt besitzt und einen Zylinder bildet.

3. Implantat nach Anspruch 1 oder 2, **dadurch gekennzeichnet, daß** die Führungsfläche (6) des ersten Implantatteils (3) an dessen Stirnfläche (7) und die Führungsfläche (6) des zweiten Implantatteils (4) in einem radial verlaufenden Schlitz (8) ausgebildet ist.

4. Implantat nach Anspruch 3, **dadurch gekennzeichnet, daß** die Führungsfläche (6) des zweiten Implantatteils (4) an der Innenseite einer Stirnplatte (9) ausgebildet ist.

5. Implantat nach Anspruch 4, **dadurch gekennzeichnet, daß** der Schlitz (8) sich radial in Richtung des ersten Implantatteils (3) erweitert, daß die Stirnplatte (9) zweiteilig mit dem zweiten Implantatteil (4) ausgebildet und in axialer Richtung verstellbar ist, und daß die Führungsfläche (6) des ersten Implantatteils (3) an einem in den Schlitz (8) eingreifenden Führungskeil (10) mit parallel zu den Schlitzwänden (8) verlaufenden Keilwänden ausgebildet ist.

6. Implantat nach Anspruch 5, **dadurch gekennzeichnet, daß** in der Stirnplatte (9) eine axial verlaufende Führungsnut (11) ausgebildet ist zum Zusammenwirken mit einem an dem zweiten Implantatteil (4) ausgebildeten Führungszapfen (12).

7. Implantat nach Anspruch 6, **dadurch gekennzeichnet, daß** der Führungskeil (10) in Umfangsrichtung als einseitig offener Ring gestaltet ist, der mit seiner Ringöffnung (13) im nicht-distrahierten Zustand den Führungszapfen (12) umgreift.

8. Implantat nach einem der Ansprüche 5 bis 7, **dadurch gekennzeichnet, daß** als Mittel zur Fixierung der relativen Lage des ersten Implantatteils (3) und des zweiten Implanttatteils (4) in der Stirnplatte (9) auf der nach innen weisenden Seite eine axial verlaufende Gewindebohrung (14) und an dem zweiten Implantatteil (4) eine Ringöse (15) ausgebildet ist zum Halten einer in die Gewindebohrung (14) eingreifenden Sicherungsschraube (16).

9. Implantat nach einem der Ansprüche 3 bis 8, **dadurch gekennzeichnet, daß** auf der der Stirnfläche (7) gegenüberliegenden Seite eine Implantatöffnung (17) für den Zugang in das Implantatinnere ausgebildet ist.

10. Implantat nach Anspruch 9, **dadurch gekennzeichnet, daß** in der Implantatöffnung (17) eine Mehrzahl von Führungsnuten (18) ausgebildet sind, von denen zwei entlang der Längsteilung zwischen dem ersten Implantatteil (3) und dem zweiten Implantatteil (4) verlaufen.

11. Implantat nach Anspruch 9 oder 10, **dadurch gekennzeichnet, daß** in dem freien Ende des Schraubenkopfes der Sicherungsschraube (16) eine Gewindebohrung (29) ausgebildet ist.

12. Implantat nach einem der Ansprüche 1 bis 11, **dadurch gekennzeichnet, daß** die in Distraktionsrichtung sich einander gegenüber liegenden Oberflächen des Rohres (5) eine Verzahnung (34) aufweisen.

13. Implantat nach einem der Ansprüche 1 bis 12, **dadurch gekennzeichnet, daß** das erste Implantatteil (3) und das zweite Implantatteil (4) aus chirurgischen Stahl und/oder Titan gebildet sind.

14. Implantat nach einem der Ansprüche 1 bis 7, **dadurch gekennzeichnet, daß** als Mittel zur Fixierung zwischen das erste Implantatteil (3) und das zweite Implantatteil (4) einzubringender Knochenzement vorgesehen ist.

15. Instrument zur Platzierung und Distraktion des Implantats nach den Ansprüchen 1 bis 14, **dadurch gekennzeichnet, daß** an einem Handgriff (20) ein hohler Stab (21) angeschlossen ist, der an seinem vorderen freien Ende ein schwenkbares Stellglied (22) aufweist, das zwei Stifte (23) zum Einsetzen in die beiden der Längsteilung zugeordneten Führungsnuten (18) aufweist, daß auf dem hohlen Stab (21) eine Hülse (24) mit einem Schwenkhebel (25) angeschlossen ist, der der Betätigung eines an dem Stellglied (22) zum Schwenken angreifenden Zugstückes (26) dient, und daß in dem hohlen Stab (21) ein hohler Schraubendreher (27) angeordnet ist, den ein Gewindestift (28) zum Einschrauben in eine Gewindebohrung (29) des Schraubenkopfes einer Sicherungsschraube (16) durchgreift.

16. Instrument nach Anspruch 15, **dadurch gekennzeichnet, daß** an dem rückwärtigen Ende des hohlen Stabes (21) ein Stabgewinde (30) ausgebildet ist mit einem darauf zur Begrenzung des Stellweges der Hülse (24) angeordneten Gewindering (31).

17. Instrument nach Anspruch 15 oder 16, **dadurch gekennzeichnet, daß** das Zugstück (26) an dem oberen Ende des Stellgliedes (22) angreift, daß an dem hohlen Stab (21) im vorderen Bereich an den gegenüberliegenden Stabwänden Nasen (32) ausgebildet sind, die eine senkrecht zur Längsteilung des Implantats (1) liegende Schwenkachse definieren, und daß das Stellglied (22) mit Abstand zu seiner Verbindung mit dem Zugstück (26) den Nasen (32) anliegt.

18. Instrument nach einem der Ansprüche 15 bis 17, **dadurch gekennzeichnet, daß** an dem Handgriff (20) ein Bügel (33) gelagert ist, der zwischen einer den axialen Verstellweg des Gewindestiftes (28) begrenzenden Stellung und einer diese Begrenzung aufhebenden Stellung verschwenkbar ist.

## Claims

1. An implant for intersomatic fusion of two adjacent vertebrae (2), comprising a first implant portion (3) and a second implant portion (4) which is displaceable relative thereto for the purposes of distraction, wherein the first implant portion (3) and the second implant portion (4) are so designed that they supplement each other in complementary relationship in the non-distracted state to afford a tube (5) which is divided in the longitudinal direction, **characterised in that** guide surfaces (6) which bear against each other and which extend radially in the distraction direction are provided on the first implant portion (3) and on the second implant portion (4), and that there are provided means for fixing the relative position of the guide surfaces (6).

2. An implant according to claim 1 **characterised in that** the tube (5) is of a circular cross-sectional configuration in the non-distracted state and forms a cylinder.

3. An implant according to claim 1 or claim 2 **characterised in that** the guide surface (6) of the first implant portion (3) is provided at the end face (7) thereof and the guide surface (6) of the second implant portion (4) is provided in a radially extending slot (8).

4. An implant according to claim 3 **characterised in that** the guide surface (6) of the second implant portion (4) is provided at the inside of an end plate (9).

5. An implant according to claim 4 **characterised in that** the slot (8) enlarges radially in the direction of the first implant portion (3), the end plate (9) is formed in two parts with the second implant portion (4) and is displaceable in the axial direction and the guide surface (6) of the first implant portion (3) is provided on a guide wedge (10) which engages into the slot (8) and which has wedge walls extending parallel to the slot walls (8).

6. An implant according to claim 5 **characterised in that** provided in the end plate (9) is an axially extending guide groove (11) for co-operation with a guide projection (12) on the second implant portion (4).

7. An implant according to claim 6 **characterised in that** in the peripheral direction the guide wedge (10) is in the form of a ring which is open at one side and which with its ring opening (13) in the non-distracted state embraces the guide projection (12).

8. An implant according to one of claims 5 to 7 **characterised in that** the means for fixing the relative position of the first implant portion (3) and the second implant portion (4) are an axially extending screwthreaded bore (14) in the end plate (9) on the inwardly facing side and a ring eye (15) on the second implant portion (4) for holding a securing screw (16) which engages into the screwthreaded bore (14).

9. An implant according to one of claims 3 to 8 **characterised in that** an implant opening (17) for access to the interior of the implant is provided on the side opposite to the end face (7).

10. An implant according to claim 9 **characterised in that** provided in the implant opening (17) are a plurality of guide grooves (18) of which two extend along the longitudinal division between the first implant portion (3) and the second implant portion (4).

11. An implant according to claim 9 or claim 10 **characterised in that** a screwthreaded bore (29) is provided in the free end of the screw head of the securing screw (16).

12. An implant according to one of claims 1 to 11 **characterised in that** the surfaces of the tube (5), which are in mutually opposite relationship in the distraction direction, have a tooth arrangement (34).

13. An implant according to one of claims 1 to 12 **characterised in that** the first implant portion (3) and the second implant portion (4) are formed from surgical steel and/or titanium.

14. An implant according to one of claims 1 to 7 **characterised in that** bone cement which is to be introduced between the first implant portion (3) and the second implant portion (4) is provided as the fixing means.

15. An instrument for the placement and distraction of the implant according to claims 1 to 14 **characterised in that** connected to a handle (20) is a hollow rod (21) which at its front free end has a pivotable setting member (22) having two pins (23) for insertion into the two guide grooves (18) associated with the longitudinal division, a sleeve (24) is connected on the hollow rod (21) to a pivot lever (25) which serves for actuating a pulling portion (26) which engages the setting member (22) for pivotal movement, and arranged in the hollow rod (21) is a hollow screwdriver (27) through which passes a screwthreaded pin (28) for screwing into a screwthreaded bore (29) in the screw head of a securing screw (16).

16. An instrument according to claim 15 **characterised in that** a rod screwthread (30) is formed at the rearward end of the hollow rod (21), with a screwthreaded ring (31) arranged on the rod screwthread for limiting the setting travel of the sleeve (24).

17. An instrument according to claim 15 or claim 16 **characterised in that** the pulling portion (26) engages the upper end of the setting member (22), noses (32) are provided at the hollow rod (21) in the front region at the oppositely disposed rod walls, the noses defining a pivot axis disposed perpendicularly to the longitudinal division of the implant (1), and the setting member (22) bears against the noses (32) at a spacing relative to its connection to the pulling portion (26).

18. An instrument according to one of claims 15 to 17 **characterised in that** mounted on the handle (20) is a stirrup (33) which is pivotable between a position limiting the axial displacement travel of the screwthreaded pin (28) and a position of removing that limitation.

## Revendications

1. Implant pour la fusion intersomatique de deux vertèbres (2) adjacentes, comportant un premier élément d'implant (3) ainsi qu'un deuxième élément d'implant (4) mobile par rapport au premier à des fins de distraction, le premier élément d'implant (3) et le deuxième élément d'implant (4) étant conformés de manière telle que ceux-ci, à l'état non distracté, se combinent de manière complémentaire pour former un tube (5) fendu dans la direction longitudinale, **caractérisé par le fait que** sur le premier élément d'implant (3) et le deuxième élément d'implant (4) sont aménagées des surfaces de guidage (6), qui s'étendent radialement dans la direction de distraction et sont en contact réciproque, et **par le fait que** des moyens sont prévus pour bloquer la position relative des surfaces de guidage (6).

2. Implant selon la revendication 1, **caractérisé par le fait que** le tube (5), à l'état non distracté, présente une section transversale circulaire et forme un cylindre.

3. Implant selon la revendication 1 ou 2, **caractérisé par le fait que** la surface de guidage (6) du premier élément d'implant (3) est aménagée sur la face frontale (7) de celui-ci et la surface de guidage (6) du deuxième élément d'implant (4) est aménagée à l'intérieur d'une fente (8) radiale.

4. Implant selon la revendication 3, **caractérisé par le fait que** la surface de guidage (6) du deuxième élément d'implant (4) est aménagée sur la face intérieure d'une plaque frontale (9).

5. Implant selon la revendication 4, **caractérisé par le fait que** la fente (8) s'élargit radialement en direction du premier élément d'implant (3), que la plaque frontale (9) est réalisée en deux parties avec le deuxième élément d'implant (4) et peut se déplacer dans la direction axiale, et que la surface de guidage (6) du premier élément d'implant (3) est aménagée sur un prisme de guidage (10), qui pénètre dans la fente (8) et dont les parois sont parallèles aux parois de la fente (8).

6. Implant selon la revendication 5, **caractérisé par le fait que** dans la plaque frontale (9) est aménagée une rainure de guidage (11) axiale qui coopère avec un tenon de guidage (12) aménagé sur le deuxième élément d'implant (4).

7. Implant selon la revendication 6, **caractérisé par le fait que** le prisme de guidage (10), dans la direction périphérique est conformé en anneau ouvert d'un côté qui, à l'état non distracté, entoure le tenon de guidage (12) avec son ouverture (13).

8. Implant selon une des revendications 5 à 7, **caractérisé par le fait qu'**il est prévu comme moyen de blocage de la position relative du premier élément d'implant (3) et du deuxième élément d'implant (4), un trou fileté (14) axial aménagé dans la plaque frontale (9), sur la face de celle-ci tournée vers l'intérieur, et un oeillet (15) aménagé sur le deuxième élément d'implant (4) afin de recevoir une vis de freinage (16) vissée dans le trou fileté (14).

9. Implant selon une des revendications 3 à 8, **caractérisé par le fait qu'**une ouverture d'implant (17) permettant d'accéder à l'intérieur de l'implant est aménagée du côté opposé à la face frontale (7).

10. Implant selon la revendication 9, ce **caractérisé par le fait que** dans l'ouverture d'implant (17) sont aménagées une pluralité de rainures de guidage (18), parmi lesquelles deux s'étendent le long de la séparation longitudinale entre le premier élément d'implant (3) et le deuxième élément d'implant (4).

11. Implant selon la revendication 9 ou 10, **caractérisé par le fait qu'**un trou fileté (29) est aménagé dans l'extrémité libre de la tête de la vis de freinage (16).

12. Implant selon une des revendications 1 à 11, **caractérisé par le fait que** les surfaces opposées tube (5), vu dans la direction de distraction, présentent une denture (34).

13. Implant selon une des revendications 1 à 12, **caractérisé par le fait que** le premier élément d'implant (3) et le deuxième élément d'implant (4) sont en acier chirurgical et/ou en titane.

14. Implant selon une des revendications 1 à 7, **caractérisé par le fait qu'**il est prévu comme moyen de blocage un ciment à os à appliquer entre le premier élément d'implant (3) et le deuxième élément d'implant (4).

15. Instrument pour la mise en place et la distraction de l'implant selon les revendications 1 à 14, **caractérisé par le fait qu'**une poignée (20) est liée à une tige (21) creuse qui, à son extrémité antérieure libre, présente un organe de positionnement (22) pivotant, pourvu de deux broches (23) qui pénètrent dans les deux rainures de guidage (18) associées à la séparation longitudinale, **par le fait que** sur la tige creuse (21), un manchon (24) est lié à un levier pivotant (25) pour l'actionnement d'un élément de traction (26) qui agit sur l'organe de positionnement (22) aux fins de faire pivoter celui-ci, et **par le fait qu'**à l'intérieur de la tige creuse (21) est disposé un tournevis creux (27) qui est traversé par une tige filetée (28) destinée à être vissée dans un trou fileté (29) de la tête d'une vis de freinage (16).

16. Instrument selon la revendication 15, **caractérisé par le fait qu'**il est prévu, à l'extrémité postérieure de la tige creuse (21), un filetage (30) sur lequel est vissée une bague filetée (31) pour limiter la course de déplacement du manchon (24).

17. Instrument selon la revendication 15 ou 16, **caractérisé par le fait que** l'élément de traction (16) agit sur l'extrémité supérieure de l'organe de positionnement (22), **par le fait que** sur la tige creuse (21), dans la région antérieure de celle-ci et sur des parois en vis-à-vis, sont aménagées des saillies (32) qui définissent un axe de pivotement normal à la séparation longitudinale de l'implant et **par le fait que** l'organe de positionnement (22) prend appui sur les saillies (32) en un point éloigné de la liaison avec l'élément de traction (26).

18. Instrument selon une des revendications 15 à 17, **caractérisé par le fait que** sur la poignée (20) est montée un étrier (33) qui peut pivoter entre une position limitant la course de déplacement axial de la tige filetée (28) et une position annulant ladite limitation.
